# EUROPÄISCHE PATENTSCHRIFT

(11) **EP 2 435 338 B1**
(45) Veröffentlichungstag und Bekanntmachung des Hinweises auf die Patenterteilung: **26.06.2013**
(21) Anmeldenummer: 10725599.4
(22) Anmeldetag: 21.05.2010
(51) Int. Cl.: B65D 81/26, A61B 19/02, B65D 77/00

(54) **Produkt mit bioresorbierbaren trägermaterialien und verpackung**
Product with bioreabsorbent carrier materials and packaging
Produit doté d'un matériau de support biorésorbant et emballage

(30) Priorität: 30.05.2009 EP 09007271
(43) Veröffentlichungstag der Anmeldung: 04.04.2012
(73) Patentinhaber: Bayer Innovation GmbH, 51373 Leverkusen (DE)
(72) Erfinder: BAECKER, Iwer, 40219 Düsseldorf (DE); BRAUN, Arne, 51375 Leverkusen (DE); GLAUBITT, Walther, 97276 Margetshöchheim (DE)
(74) Vertreter: BIP Patents
(86) Internationale Anmeldenummer: PCT/EP2010/003146
(87) Internationale Veröffentlichungsnummer: WO 2010/139407

(56) Entgegenhaltungen:
- FR-A- 2 660 634
- GB-A- 2 103 565
- US-A- 5 322 161
- US-B1- 6 269 946

## Beschreibung

Die vorliegende Erfindung betrifft ein Produkt mit bioresorbierbaren Trägermaterialen insbesondere für die Geweberegeneration und eine Verpackung, sowie Verfahren zur Herstellung des Produkts.

Bei bioresorbierbaren Trägermaterialien zur Geweberegeneration, wie beispielsweise Wundauflagen aus Kieselgelfasern bzw. Vliesen, wurde festgestellt, dass über Lagerzeiträume im Bereich mehrerer Monate oder Jahre die Biodegradationseigenschaften der Trägermaterialien negativ beeinflusst werden. Es hat sich insbesondere gezeigt, dass bioresorbierbare Trägermaterialien, die in herkömmlichen Verpackungen gelagert wurden, eine verzögerte Bioresorbierbarkeit bzw. eine unvollständige Bioresorbierbarkeit aufweisen. Letzteres kann dazu führen, dass Rückstände im Gewebe zurückbleiben, die über längere Zeit Entzündungsreaktionen hervorrufen und zu einer nicht physiologischen Wundheilung führen können. Gegenstand der vorliegenden Erfindung ist, dass nun überraschend gefunden wurde, dass die Biodegradationseigenschaften der bioresorbierbaren Trägermaterialien durch geeignete Verpackungsmaterialien über einen langen Zeitraum gewährleistet werden können. Dies ist insbesondere im Zusammenhang mit der Lagerung von bioresorbierbaren Trägermaterialen zur Geweberegeneration, wie beispielsweise bioresorbierbaren Wundauflagen aus Kieselgel von besonderer Bedeutung.

Wundauflagen aus bioresorbierbarem Trägermaterial werden für die Behandlung schwer heilender Wunden, wie sie z. B. bei Diabetes-bedingten Ulcern, Dekubitus, Brandverletzungen oder auch chirurgischen Wunden vorliegen, vorgeschlagen. Hierbei ist das Resorptionsverhalten des Materials während des Heilungsprozesses von zentraler Bedeutung für den Behandlungserfolg. Wundauflagen auf Kieselgelfaserbasis eignen sich unter anderem aufgrund ihrer Bioresorptionseigenschaften und ihrer Bioverträglichkeit vorzüglich zum Einsatz in der Heilung schwer heilender Wunden. Je nach Applikationstyp und Art der Wunde wird ein spezifisches Resorptionsverhalten benötigt, das über die Lagerzeit des Produktes eingehalten werden muss. Neben der Aufrechterhaltung der Biodegradationseigenschaften ist die Gewährleistung der Stabilität durch einen mechanischem Schutz der Wundauflagen während der Lagerung und dem Transport ein weiterer wichtiger Aspekt, der bei der Bereitstellung einer geeigneten Verpackung beachtet werden muss. Äußere Einwirkungen, wie sie beispielsweise beim Transport auftreten, können Relativbewegungen der Wundauflage zur Verpackung auslösen, die ein Ausfransen der Ecken und Kanten bewirken und zu Faserablösung oder Faserbruch führen, der aus ästhetischen und praktischen Gründen bei der Applikation unerwünscht ist. Daher wird eine Form benötigt, die Relativbewegungen in alle Richtungen auf ein Mindestmaß einschränkt. Dabei ist aufgrund der Kompressibilität des Materials - insbesondere bei größeren Abmessungen der Wundauflage im Bereich 10 cm * 10 cm oder mehr - eine mechanische Fixierung, die eine Flächenpressung auf das Material bewirkt, wie sie z. B. durch Vakuum in einem flexiblen System erreicht werden könnte, möglichst gering zu halten bzw. zu vermeiden. Weiterhin ist bei einer einfachen Verpackung im Verbundfolienbeutel nicht ausreichend Schutz gegenüber mechanischer Einwirkung von außen, die zu einer Schädigung der Wundauflage führen kann, gegeben. Verpackungen zum Lagen von bioresorbierbaren Trägematerial sind aus US 5 322 161 und GB 2 103 565 bekannt. Es besteht ein Bedarf nach Produkten mit bioresorbierbaren Trägermaterialien insbesondere für die Geweberegeneration, die den Verlust der Bioresorptionseigenschaften der bioresorbierbaren Trägermaterialien verhindern können. Gleichzeitig müssen die Produkte die bioresorbierbaren Trägermaterialien ausreichend gegenüber mechanischer Einwirkung absichern.

Die Aufgabe der vorliegenden Erfindung war es demnach, Produkte mit bioresorbierbaren Trägermaterialen insbesondere für die Geweberegeneration bereitzustellen, die die bestehenden Nachteile adressieren und die oben genannten Anforderungen bzgl. der Gewährleistung der Biodegradationseigenschaften und des mechanischen Schutzes erfüllen. Erfindungsgemäß wird die Aufgabe durch die Bereitstellung eines Produkts gema̋ß Anspruch 1 umfassend:
a) eine Verpackung aus
   1) einem mechanisch stabilen Basiselement (1) mit wenigstens einer nach wenigstens einer Seite hin offenen Kavität (2a), zur Entnahme wenigstens eines in der Kavität (2a) angeordneten bioresorbierbaren Trägermaterials,
   2) zumindest einem Adsorbens,
   3) zumindest einer Siegelfolie (3), die zumindest die Öffnung der Kavität, welche ein darin eingebettetes bioresorbierbares Trägermaterial enthält, verschließt und
   4) optional einem Beutel (4) aus Aluminium- oder Aluminiumverbundfolie, welcher das Basiselement (1) mit Siegelfolie (3) komplett umhüllt und verschließt,
b) ein bioresorbierbares Trägermaterial, welches sich zumindest in einer Kavität (2a) des mechanisch stabilen Basiselements (1) der Verpackung befindet, gelöst.

Das erfindungsgemäße Produkt zeichnet sich überraschenderweise durch eine Gewährleistung der Biodegradationseigenschaften der bioresorbierbaren Trägermaterialen, Stabilität gegenüber äußeren mechanischen Einwirkungen und/oder Bewegungen, und einfache Handhabung bei der Applikation aus.

Im Sinne der vorliegenden Erfindungen bezeichnet der Ausdruck "bioresorbierbar" bzw. "biodegradierbar" die Eigenschaft des Trägermaterials abgebaut zu werden, wenn das Trägermaterial Bedingungen ausgesetzt wird, die typisch sind für die diejenigen, die bei einer Geweberegeneration (beispielsweise einer Wunde, bzw. Knorpel oder Knochenmilieu) vorliegen. "Bioresorbierbar" bzw. "biodegradierbar" im Sinne der Erfindung sind die bioresobierbaren bzw. biodegradierbaren Trägermaterialien insbesondere dann, wenn sie sich nach 48 Stunden, vorzugsweise 36 Stunden und besonders bevorzugt nach 24 Stunden in einer 0,05 M Tris pH 7,4 Pufferlösung (Fluka 93371) thermostatisiert bei 37°C vollständig lösen (siehe Beispiel 3).

Geeignete bioresorbierbare Trägermaterialien (ohne darauf begrenzt zu sein) sind beispielsweise Kieselgel, Polyglycolsäure, Polylactidsäure, Polydioxanon, Polylactidcoglycolid, Poly-ε-caprolacton, Polyanhydrid, Polyphosphazen, Polyorthoester, Alginat, Chondroitin-6-sulfat, Chitosan, Hyaluronsäure, Kollagen, Polylysin, Dextran, Heparin etc. Bevorzugte bioresorbierbare Trägermaterialien sind solche, die für die Geweberegeneration eingesetzt werden können. Unter dem Begriff "Trägermaterialien zur Geweberegeneration" werden Flächenelemente und/oder dreidimensionale Raumelemente verstanden, die aus einem bioresorbierbaren Material bestehen und die Geweberegeneration unterstützen. Das bioresorbierbare Material kann für die Regeneration eines beliebigen Gewebes wie beispielsweise Epithel, Endothel, Urothel, Mukosa, Dura, Bindegewebe etc. eingesetzt werden. So kann beispielsweise für die Wundheilung als bioresorbierbares Trägermaterial ein Flächenelement (Wundauflage) eingesetzt werden. Für die Knorpel- oder Knochenregeneration wird ein Flächenelemente und/oder ein dreidimensionale Raumelement verwendet. Bevorzugte bioresorbierbare Trägermaterialien sind solche die auf der Basis von Kieselgel beruhen. Kieselgel-Trägermaterialen können Kieselgelfasern, Vliese, Monolithe, Hydrogele und/oder Pulver umfassen. Erfindungsgmäß sind bioresorbierbare Trägermaterialien auf Basis von Kieselgelfasern und/oder Kieselgelvliesen. Besonders bevorzugt sind Wundauflagen auf Basis von Kieselgelfasern und/oder Kieselgelvliesen wie sie beispielsweise in der DE 102004063599B4 oder der WO2008086970A1 beschrieben sind. Weitere bioresorbierbare Trägermaterialien auf Basis von Kieselgel sind Hautimplantate wie sie aus der WO200142428A1 bekannt sind oder Zellverbunde, Leitstrukturen, Gewebe oder Composite grafts wie sie in der EP1262542A2 beschrieben sind.

Das erfindungsgemäße mechanisch stabile Basiselement (1) besteht aus üblichen Kunststoffen wie etwa Polyethylenterephthalat (PETG), Polyvinylchlorid (PVD), COC (Cycloolefin- Copolymer, z.B. Topas^{®}, Cycloolefinpolymer (COP), Polychlorotrifluorethylen (z.B. ACLAR^{®}), Polyethylen (PE), Polypropylen, Polyvinylidenchloride (PVDC), Polycarbonat, Polyester, Polyacrylat, Polyamid oder anderen Kunststoffen.

Das mechanisch stabile Basiselement kann optional eine Metallfolie aus Aluminium- oder einer Aluminiumverbundfolie umfassen. Bei dieser Ausführungsform der Erfindung wird beispielsweise eine tiefgezogene Aluminiumschale mit einer Aluminiumverbundfolie gegebenenfalls unter leichtem Vakuum mit einer Aluminiumverbundfolie versiegelt. Die Aluminiumschale ist dabei innen (d.h. auf derjenigen Seite, wo das bioresorbierbare Trägermaterial bzw. das Adsorbens aufgenommen wird) mit einem Siegellack und/oder einer Beschichtung auf Polymerbasis versehen. Auf der Außenseite kann die Aluminiumschale optional durch einen Kunststoffblister verstärkt werden. Die Siegelfolie (3) ist bei dieser Ausführungsform der Erfindung auch vorzugsweise eine Aluminium- oder eine Aluminiumverbundfolie.

Die mechanische Stabilität eines Basiselements ist gewährleistet, wenn die Biegesteifigkeit sowie die Stabilität gegenüber Knicken so hoch ist, dass beim Abziehen der aufgesiegelten Folie die Form des Basiselementes weitestgehend erhalten bleibt, so dass der Anwender das bioresorbierbare Trägermaterial und insbesondere eine Wundauflage sicher entnehmen kann und z.B. ein Herausfallen bei geübter Handhabung ausgeschlossen ist. Die Steifigkeit kann durch z.B. Wahl der Materialstärken wie auch durch Profilierung des Basiselementes (1) beim Tiefziehen gewährleistet werden. Das Basiselement stellt eine Fläche dar, in der mindestens eine Kavität ausgebildet ist. In einer bevorzugten Ausführungsform der Erfindung umfasst das mechanisch stabile Basiselement (1) eine zweite Kavität (2b) zur Aufnahme eines Adsorbens. Beim Vorhandensein einer zweiten Kavität verschließt die Siegelfolie (3) vorzugsweise die Öffnungen beider Kavitäten (2a und 2b).

Die Flächen des Basiselements sind vorzugsweise, sieht man von den Kavitäten ab, plan. Die Öffnungen der Kavitäten sind bevorzugt auf der gleichen Seite des Basiselements. Ein planares Basiselement kann eine beliebige geometrische Form einnehmen. Vorzugsweise ist das Basiselement viereckig. Das Basiselement kann auch die Funktion einer Sterilbarriere übernehmen. Bei der bevorzugten Ausführungsform der Erfindung sind die mindestens zwei Kavitäten des Basiselements in einer Reihe, parallel, kreisförmig, spiralförmig oder in mehreren Reihen angeordnet und können eine beliebige geometrische Form, wie beispielsweise rund, viereckig, sechseckig etc. einnehmen. Vorzugsweise ist die Kavität zur Aufnahme des bioresorbierbaren Trägermaterials und hier insbesondere der Wundauflage viereckig. In einer bevorzugten Ausführungsform enthält die Kavität (2a) zur Aufnahme der Wundauflage eine weitere Aussparung (11), welche die Entnahme der Wundauflage erleichtert, jedoch die mechanische Fixierung der Wundauflage in der Kavität (2b) nicht beeinflusst. Die Aussparung (11) ist bei dieser Ausführungsform der Erfindung derart ausgestaltet, dass sie nur einen Teilbereich einer Kavitätswand (10) einnimmt.

Die Kavität (2b) zur Aufnahme des Adsorbens kann offen oder geschlossen sein. Im letzteren Fall wird das Basiselement während der Herstellung so geformt, dass es selbst einen abgeschlossenen Hohlraum mit einem darin enthaltenen Adsorbensmaterial ausbildet. Zwischen der Kavität (2a) und der Kavität (2b) ist ein Steg (9), der durch das Basiselement gebildet wird, vorhanden. Der Steg kann auch nur teilweise ausgebildet sein, so dass beispielsweise die Siegelfolie (3) beim Verschluss der Kavitäten (2a) und (2b) nicht mit dem Steg (9) in Berührung kommt.

Überraschend wurde gefunden, dass durch die Zugabe mindestens eines Adsorbens zur erfindungsgemäßen Verpackung die Biodegradierbarkeit des Trägermaterials über einen langen Zeitraum gewährleistet werden kann. Im Falle von Trägermaterialien auf Basis von Kieselgel handelt es sich bei dem mindestens einen Adsorbens um ein Adsorbens für Wasser und Ethanol.

Vorzugsweise werden ein oder mehrere Adsorbentien für Wasser und für Ethanol eingesetzt. Adsorbentien für Wasser und/oder Ethanol sind aus der Literatur bekannt. Für Wasser eignen sich beispielsweise Silicagele und/oder Zeolithen mit einem Porendurchmesser im Bereich von 2-5 nm (Hinkle, L. D., "Effect of purge pressure on desorbing water removal rate" Journal of Vacuum Science and Technology A: Vacuum, Surfaces and Films 2004, 22 (4), 1799-1803 / Gorbach, A.; Stegmaier, M.; Eigenberger, G., "Measurement and Modeling of Water Vapor Adsorption on Zeolite 4A-Equilibria and Kinetic." Adsorption 2004, 10 (1), 29-46 / Grubner, O. et al., "Molekularsiebe", Berlin, Deutscher Verlag der Wissenschaften 1968). Beispielhaft seien Silicagel, Zeolith 3A und Zeolith 4A genannt.

Als Adsorbentien für Ethanol eignen sich beispielsweise Silikalit-1, F-Silikalit und ZSM-5 (Cekova Blagica et al., "Zeolites as alcohol adsorbents from aqueous solutions", Acta periodica technologica, iss. 37, pp. 83-87, 2006).

Mittels Silicagel als Adsorbens in der erfindungsgemäßen Verpackung kann eine Biodegradierbarkeit eines Trägermaterials auf Basis von Kieselgel für einen Lagerzeitraum von mehreren Wochen erreicht werden (siehe Beispiel 3). Silicagel ist dabei als Adsorbens für Wasser und Ethanol geeignet. Ethanol und Wasser unterscheiden sich unter anderem in ihrer Molekülgröße. Ein speziell für Ethanol geeignetes Adsorbens hat daher einen auf die Molekülgröße von Ethanol angepassten Porendurchmesser, während ein speziell für Wasser geeignetes Adsorbens einen auf die Molekülgröße von Wasser angepassten Durchmesser aufweist.

Überraschend hat sich gezeigt, dass durch Zugabe von zwei verschiedenen Adsorbentien zu der erfindungsgemäßen Verpackung, von denen eines speziell für Wasser geeignet ist und das andere speziell für Ethanol geeignet ist, ein Produkt erhalten wird, bei dem eine Biodegradierbarkeit über mehrere Monate gewährleistet ist (siehe Beispiel 3).

Das erfindungsgemäße Produkt umfasst daher mindestens zwei Adsorbentien, wobei mindestens eines der Adsobentien ein Adsorbens für Wasser (wie beispielsweise Zeolith 3A oder Zeolith 4A) und mindestens eines der Adsorbentien ein Adsorbens für Ethanol (wie beispielsweise Silikalit-1) ist.

Ein oder mehrere Adsorbentien können innerhalb (d.h. beispielsweise in der dafür vorgesehenen Kavität oder an einer anderen Stelle innerhalb des Basiselementes) und/oder außerhalb (d.h. im Beutel, jedoch nicht in der dafür vorgesehenen Kavität) des Basiselementes eingebracht werden. Vorzugsweise wird das Adsorbens / werden die Adsorbentien in die dafür vorgesehene Kavität des Basiselements eingearbeitet. Hierdurch wird die Handhabung vereinfacht, da das Adsorbens nicht aus dem Beutel herausfallen kann. Ein oder mehrer Adsorbentien können bei anderen Ausführungsformen aber beispielsweise auch als Monolith in die Kavität (2a) hineingegeben werden und durch eine permeable Folie abgedeckt werden. Auf diese Folie wird dann das bioresorbierbare Trägermaterial (beispielsweise eine Wundauflage) gegeben. Alternativ können ein oder mehrere Adsorbentien auch in einem kleinen Beutel (Pouch) eingebracht werden und in dieser Form unter die aufzusiegelnde Aluminiumverbundfolie d.h. die Siegelfolie (3) gesiegelt werden.

Das Mengenverhältnis zwischen dem bioresorbierbaren Trägermaterial auf Basis von Kieselgel und dem Adsorbensmaterial ist vorzugsweise zwischen 2:1 und 1:20 und besonders bevorzugt zwischen 1:2 und 1:10.

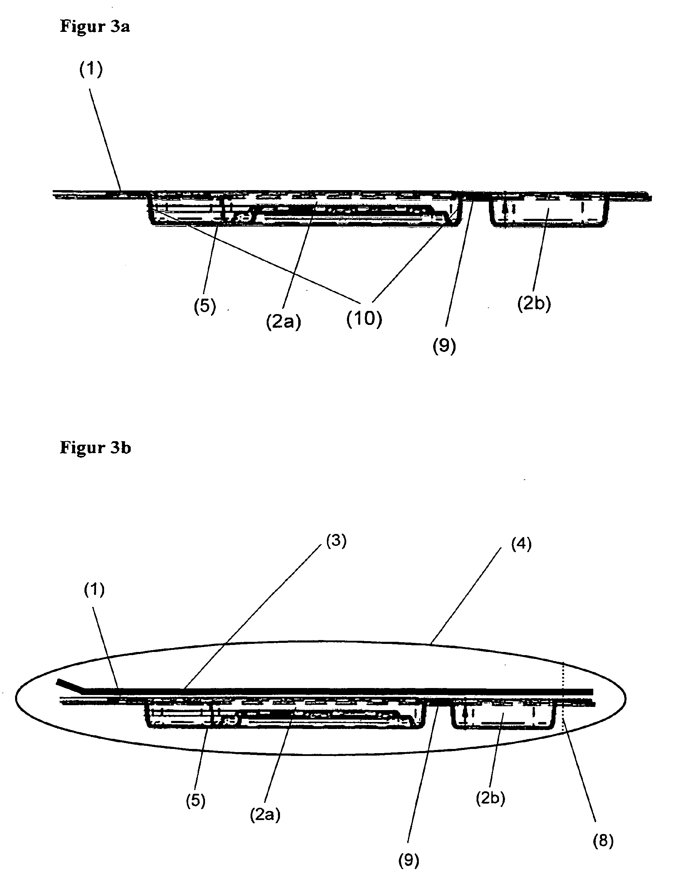

Erfindungsgemäß wird zumindest die Öffnung der Kavität des Basiselements, welche ein darin eingebettetes resorbierbares Trägermaterial enthält, mit einer Siegelfolie (3) verschlossen (durch Heißversiegeln der Folie auf dem Basiselement). Die Siegelfolie verhindert das Eindringen von Partikeln und ermöglicht z.B. beim Vorhandensein eines Beutels (4) eine Entnahme der bioresorbierbaren Trägermaterialien, ohne dass diese bereits beim Öffnen des Beutels (4) herausfallen können. Die Siegelfolie ist eine Metall- und/oder Polymerfolie. Die Siegelfolie kann aber auch selbst ein Beutel aus Aluminium- oder Aluminiumverbundfolie sein. In diesem Fall kann die Siegelfolie beispielsweise die Öffnungen des Basiselements derart verschließen, dass das Basiselement, das Adsorbens und das bioresorbierbare Trägermaterial von der Siegelfolie (als Beutel) komplett umhüllt sind. Bei dieser Ausführungsform muss die Siegelfolie nicht mit dem Basiselement beispielsweise durch Heißsiegeln verbunden werden, sondern kann z.B. unter leichtem Vakuum mit sich selber verschlossen (d.h. versiegelt) werden. Die Siegelfolie (3) kann aus den gleichen Materialien wie das Basiselement gefertigt sein. Als Metallfolie wird beispielsweise eine Aluminium- oder Aluminiumverbundfolie verwendet. Als Polymerfolie kann beispielsweise eine Folie auf Tyvek-Basis (DuPont) benutzt werden. Die Aluminiumschicht der Aluminium- oder Aluminiumverbundfolien der vorliegenden Erfindung haben bevorzugt eine Schichtdicke von mindestens 10µm, besonders bevorzugt mindestens 16 µm. Die Aluminium- oder Aluminiumverbundfolie besteht in einer bevorzugten Ausführungsform der Erfindung aus mehreren Schichten. Beispielsweise ist die Folie dreilagig und besteht von Außen nach Innen aus PET, Aluminium und PE. In einer weiteren bevorzugten Ausführungsform der Erfindung umhüllt ein zusätzlicher Beutel aus Aluminium- oder Aluminiumverbundfolie (4) das Basiselement (1) mit Siegelfolie (3) sowie das Adsorbens und das sich in der Kavität des Basiselements befindliche bioresorbierbare Trägermaterial komplett und verschließt dieses (z.B. durch Heißversiegeln mit sich selber). In einer besonders bevorzugten Ausführungsform der Erfindung besteht der Beutel aus mindestens zwei verschiedenen Schichten, bei denen die Verbindung zwischen der inneren Schicht (d.h. derjenigen Schicht, die mit dem Basiselement und der Siegelfolie in Kontakt kommt) und der Aluminiumfolie durch Heißlaminierung erfolgt. Hierbei kann auf den Einsatz eines Klebstoffs verzichtet werden. Alternativ sind Klebstoffe oder Haftmittel bevorzugt, bei deren Herstellung keine Isocyanate eingesetzt worden sind. Spuren von z. B. Methylendiisocyanat (MDI) oder Toluendiisocyanat (TDI) können in Zusammenhang mit Nebenkomponenten in der Kieselgelfaser unerwünschte Verfärbungen hervorrufen. Verbundfolien der beschriebenen Ausführung sind auf dem Markt beispielsweise von Amcor erhältlich. Der Beutel wird verschlossen, so dass sich das Basiselement mit Siegelfolie im Beutel befinden. In einer bevorzugten Ausführungsform wird der Folienbeutel bzw. ein Teil davon zur leichten Öffnung durch den Anwender abziehbar oder mit einem Zipper ausgestaltet (in Figur 3b dargestellt durch (8)).

Eine bevorzugte Ausführungsform der vorliegenden Erfindung betrifft ein Produkt umfassend eine Verpackung, bei der die Kavität (2a) des mechanisch stabiles Basiselement (1) zur Entnahme wenigstens einer in der Kavität (2a) angeordneten Wundauflage, in der Höhe, Breite und Länge so ausgestaltet ist, dass ein Verrutschen der Wundauflage in der Kavität in eine der drei Dimensionen im Wesentlichen verunmöglicht wird. Grundprinzip ist hierbei, dass durch enge Spaltweiten zwischen Rand der Kavität und Wundauflage die Relativgeschwindigkeit zwischen Wundauflage und Blister bei äußerer mechanischer Einwirkung (z. B. Erschütterung) eng begrenzt wird; so dass beim Stoß der Wundauflage gegen das Blister die übertragene Energie so gering ist, dass es auch bei wiederholter Beanspruchung zu keiner Produktschädigung kommt. Demnach wird zum mechanischen Schutz der Wundauflage die Abmessung der Kavität des Basiselements zur Aufnahme der Wundauflage eng an die Abmessungen der Wundauflage angepasst. Besonders bevorzugt ist ein seitlicher Randabstand der Wundauflage zu jeweils einem der Kavitätswände (10) von weniger als etwa 1 mm, ganz besonders bevorzugt von weniger als etwa 0,5 mm. Hierdurch wird ein seitliches Verrutschen verhindert, das insbesondere in den Ecken zum Ausfransen führen kann. Die Höhe der Kavität (von der Bodenplatte (5) , bzw. von einem Steg (7) zu der Siegelfolie) zur Aufnahme der Wundauflage übersteigt die Dicke der Wundauflage um weniger als etwa 5 mm, besonders bevorzugt um weniger als etwa 2 mm. Dadurch wird die Wundauflage in alle 3 Dimensionen (Höhe, Breite, Länge) in der Kavität mechanisch fixiert. Die Wundauflage hat vorzugsweise folgende Dimensionen 2,5*2,5 cm bis 20*20cm. Illustrativ ist demnach - bei einer 10*10cm Wundauflage die Kavität zur Aufnahme der Wundauflage folgendemaßen ausgestaltet: Länge und Breite der Kavität vorzugsweise jeweils etwa 10,01 - 10,1 cm; Höhe entspricht der Dicke der Wundauflage plus zusätzlich vorzugsweise 0,3 mm bis 5 mm.

Das Risiko des Ausfransen der Wundauflage kann weiter verringert werden, indem die Wundauflage aus dem Vliesgelege derart ausgestanzt wird, dass runde oder eckige Formteile entstehen. Bei eckigen Formteilen wie beispielsweise Viereck oder Sechseck, werden die Stanzmesser so ausgeführt, dass die Ecken der Wundauflage abgerundet sind.

In einer weiteren bevorzugten Ausführungsform wird das erfindungsgemäße Produkt umfassend eine Verpackung derart ausgestaltet, dass die Kavität (2a) des mechanisch stabilen Basiselements (1) zur Entnahme wenigstens einer in der Kavität (2a) angeordneten Wundauflage eine Bodenplatte (5) mit Kanälen (6) zur Aufnahme von Benetzungsflüssigkeit und Stege (7) zur Drainage der Benetzungsflüssigkeit bei der Entnahme aufweist. Durch die Ausgestaltung der Bodenplatte mit Kanälen und Stegen, kann eine hydrophobe Wundauflage vor der Entnahme (z.B. mit einer Pinzette) mit einer (isotonischen) Salz- oder Pufferlösung benetzt werden. Dadurch wird eine vereinfachte (komplikationsfreie) Applikation der Wundauflage direkt auf die Haut ermöglicht und ein schneller und weitgehender Austausch mit dem Wundgewebe und -exsudat gewährleistet. Der Abstand zwischen Stegen, d.h. die Kanalbreite ist mindestens so groß, dass ihre Benetzung mit der Benetzungsflüssigkeit erfolgt und diese einfach abfließen kann. Die Kanalbreite ist dabei so klein zu wählen, dass die benetzte Wundauflage zwecks Handhabbarkeit nicht zwischen Stegen durchhängt. Die Breite der Stege ist vorzugsweise so gewählt, dass eine ausreichende Abstützung der Wundauflage gewährleistet ist. Weitere Aspekte bei der Wahl der Anzahl und Abmessungen der Stege bzw. Kanäle sind die Tiefziehbarkeit des jeweiligen Materials sowie ästhetische Gesichtspunkte.

Eine weitere bevorzugte Ausführungsform betrifft das erfindungsgemäße Produkt umfassend eine Verpackung bei der die Kavität (2b) des mechanisch stabiles Basiselement (1) zur Aufnahme eines Adsorbens auf mindestens einer Seite des Basiselement offen ist und die Siegelfolie die Öffnungen der wenigstens zwei nach wenigstens einer Seite hin offenen Kavitäten (2a und 2b) des Basiselements unter Ausbildung von Hohlräumen verschließt und sich die Siegelfolie von einem Anwender nur zur Entnahme wenigstens eines in der Kavität (2a) angeordneten bioresorbierbaren Trägermaterials abziehen lässt. Bei der Integration des Adsorbens in eine Kavität (2b) des Basiselements soll ein Kontakt mit einer Benetzungsflüssigkeit vermieden werden. Dazu soll diese (zweite) Kavität (2b) mit einer Siegelfolie verschlossen und auch beim Öffnen der Kavität (2a), welche das bioresorbierbare Trägermaterial zur Geweberegeneration enthält, nicht geöffnet werden.

In einer weiteren erfindungsgemäß bevorzugten Ausgestaltungsform befindet sich deshalb auf dem Steg (9) zwischen der Siegelfolie, welche die Kavität mit dem bioresorbierbaren Trägermaterial (2a) und derjenigen, welche die Kavität mit dem Adsorbens (2b) verschließt, eine Aussparung. Alternativ kann durch Perforation der auf den Steg (9) versiegelten Siegelfolie eine definierte Abrisskante geschaffen werden. Somit bleibt die das Adsorbens aufnehmende Kavität des Basiselements bei Öffnen der Siegelfolie verschlossen.

In einer alternativen Ausführungsform der Erfindung wird das Adsorbensmaterial derart in die Kavität des Basiselements integriert, dass es vollständig vom Basiselement umhüllt ist und nicht mehr von einer Siegelfolie verschlossen werden muss. Die Kavität zur Aufnahme des Adsorbens bildet in dieser Ausführungsform der Erfindung einen durch das Basiselement selber geformten abgeschlossenen Hohlraum.

Die vorliegende Erfindung betrifft des Weiteren auch ein Verfahren zur Herstellung eines erfindungsgemäßen Produkts bei der
a) in eine Verpackung mit einem mechanisch stabilen Basiselement (1) mit wenigstens einer nach wenigstens einer Seite hin offenen Kavitäten (2a), zur Entnahme wenigstens eines in der Kavität (2a) bioresorbierbaren Trägermaterials wenigstens ein bioresorbierbares Trägermaterial in die Kavität (2a) eingelegt wird,
b) eine Siegelfolie (3) zumindest diejenige Kavität, welche ein darin eingebettetes bioresorbierbares Trägermaterial enthält, derart verschlossen wird, dass entweder zwischen der Siegelfolie (3) und einem Teil des Basiselements (1) eine die Kavität vollständig umschließende heißversiegelte Verbindung entsteht oder zwischen Teilen der Siegelfolie (3) selber eine heißversiegelte Verbindung entsteht, so dass das Basiselement und das bioresorbierbare Trägermaterial sich nach dem Verschließen in der Siegelfolie (3) befinden,
c) optional das mechanisch stabile Basiselement mit der durch die Siegelfolie (3) verschlossenen Kavität mit einem Beutel (4) aus Aluminium- oder Aluminiumverbundfolie, welcher das Basiselement (1) mit Siegelfolie (3) komplett umhüllt, versiegelt wird und
d) mindestens ein Adsorbens, für Wasser und mindestans ein Adsorbens für Ethanol, vor dem Versiegeln in Schritt b) oder c) zur Verpackung dazugegeben wird.

Falls bei mechanisch stabilen Basiselement (1) eine zweite Kavität (2b) vorhanden ist, so verschließt die Siegelfolie (3) vorzugsweise beide Kavitäten. Das mindestens eine Adsorbens wird dann vor dem Heißsiegeln mit der Siegelfolie (3) in die Kavität (2b) eingelegt. Alternativ kann das mindestens eine Adsorbens auch in einem kleinen Beutel (Pouch) eingebracht werden und in dieser Form unter die Siegelfolie (3) gesiegelt werden. Bei dieser Ausführungsform kann auch nur eine Kavität (2a) vorhanden sein solange genügend Platz für das bioresorbierbare Trägermaterial und das mindestens eine Adsorbens vorhanden ist. Auch bei dieser Ausführungsform muss das mindestens eine Adsorbens noch vor dem Heißsiegeln in die Kavität eingebracht werden. Alternativ kann das Adsorbens in den Beutel (4) aus Aluminium- oder Aluminiumverbundfolie vor dem Versiegeln des Beutels dazugegeben werden. In einer weiteren Ausführungsform der Erfindung werden die Kavitäten des Basiselements vor der Siegelung der Siegelfolie (3) mit einem wasserfreien Inertgas (wie beispielsweise Stickstoff) gespült.

Falls die Verpackung derart ausgestaltet ist, dass die Kavität (2b) zur Aufnahme eines Adsorbens auf mindestens einer Seite des Basiselement offen ist und die Siegelfolie (3) die Öffnungen der wenigstens zwei nach wenigstens einer Seite hin offenen Kavitäten (2a und 2b) des Basiselements unter Ausbildung von Holräumen verschließt und sich die Siegelfolie von einem Anwender nur zur Entnahme wenigstens eines in der Kavität (2a) angeordneten bioresorbierbaren Trägermaterials abziehen lassen soll, dann können bei der Herstellung beispielsweise zwei getrennte Siegelfolien für Kavität (2a) und für (2b) heißgesiegelt werden. In einer weiteren Ausführungsform kann auch nur eine Siegelfolie (3) aufgebracht werden, die dann auf dem Steg (9) perforiert wird, so dass eine definierte Abrisskante entsteht.

Als mechanisch stabiles Basiselement werden erfindungsgemäß entweder vorgefertigte Blister (Basiselement mit Kavität(en)) eingesetzt oder die Blister werden aus einer Kunststofffolie an einer Tiefziehmaschine tiefgezogen. Danach wird ein bioresorbierbares Trägermaterial, vorzugsweise eine Wundauflage, und ein Adsorbens in die dafür vorgesehene Kavitäten des Basiselements eingelegt. Anschließend wird die Siegelfolie aufgebracht und die Folie mit dem Blister durch Heißsiegeln verbunden. Der geschlossene Blister wird vorzugsweise in einen vorgefertigten Aluminium- oder Aluminiumverbundfolienbeutel eingebracht und die letzte Naht mittels Siegelmaschine heißversiegelt. Alternativ kann der Beutel in einer 4-Seiten-Siegelmaschine aus einer Aluminium- oder Aluminiumverbundfolie hergestellt werden. Die weitere Verpackung z. B. in Faltschachteln, die Etikettierung, Zugabe von Beipackzetteln etc. erfolgen analog dem bekannten Stand der Technik.

Die vorliegende Erfindung betrifft auch eine Verwendung der in dieser Anmeldung beschriebenen Verpackung zum verpacken und lagern von den in dieser Anmeldung beschriebenen bioresorbierbaren Trägermaterialien.

Die Erfindung soll mit folgenden Figuren und Beispielen näher erläutert werden, ohne hierauf beschränkt zu sein.

### Figuren

### Legende:

- (1):: Basiselement
- (2a):: Kavität (2a) zur Entnahme wenigstens eines in der Kavität (2a) angeordneten bioresorbierbaren Trägermaterials
- (2b):: Kavität (2b) zur Aufnahme eines Adsorbens
- (3):: Siegelfolie
- (4):: Beutel
- (5):: Bodenplatte der Kavität (2a)
- (6):: Kanal bzw. Kanäle zur Aufnahme von Benetzungsflüssigkeit
- (7):: Steg zur Drainage der Benetzungsflüssigkeit
- (8):: Zipper bzw. Element, welches das Öffnen des Beutels (4) erleichtert
- (9):: Steg zwischen der Kavität (2a) und der Kavität (2b)
- (10):: Kavitätswand bzw. Kavitätswände
- (11):: Aussparung zur vereinfachten Produktentnahme aus der Kavität (2a)

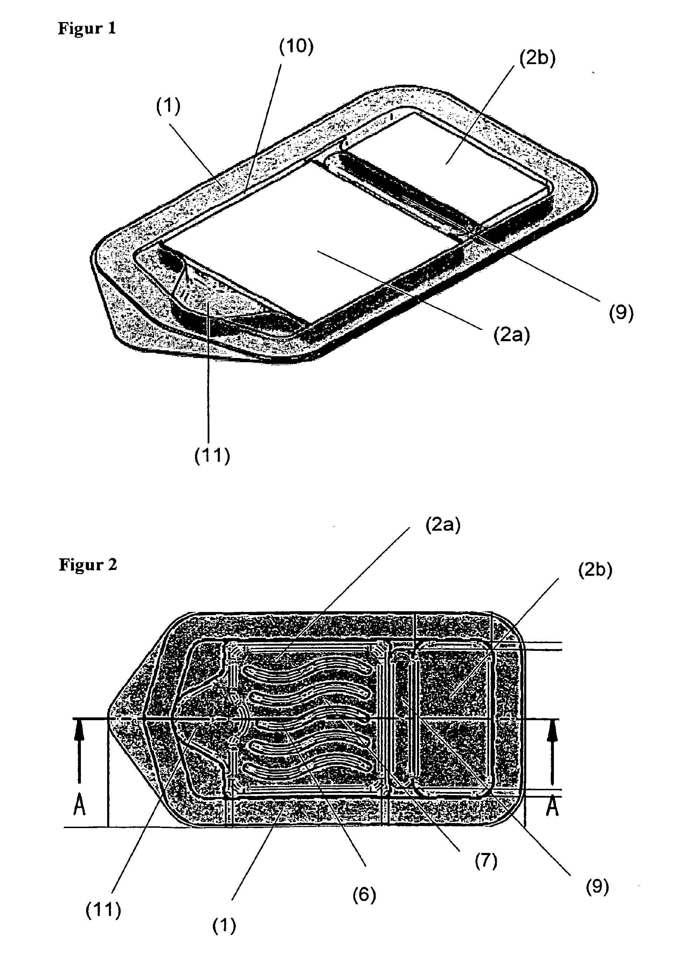

- Figur 1 :: zeigt ein Beispiel eines Basiselements mit Kavitäten von unten
- Figur 2:: zeigt ein Beispiel eines Basiselements mit Kavitäten in der Draufsicht dargestellt
- Figur 3a:: zeigt ein Beispiel eines Basiselements mit Kavitäten von der Seite
- Figur 3b:: zeigt ein Beispiel eines Basiselements mit Kavitäten, der Siegelfolie (3) sowie dem optional vorhandenen Beutel (4) von der Seite

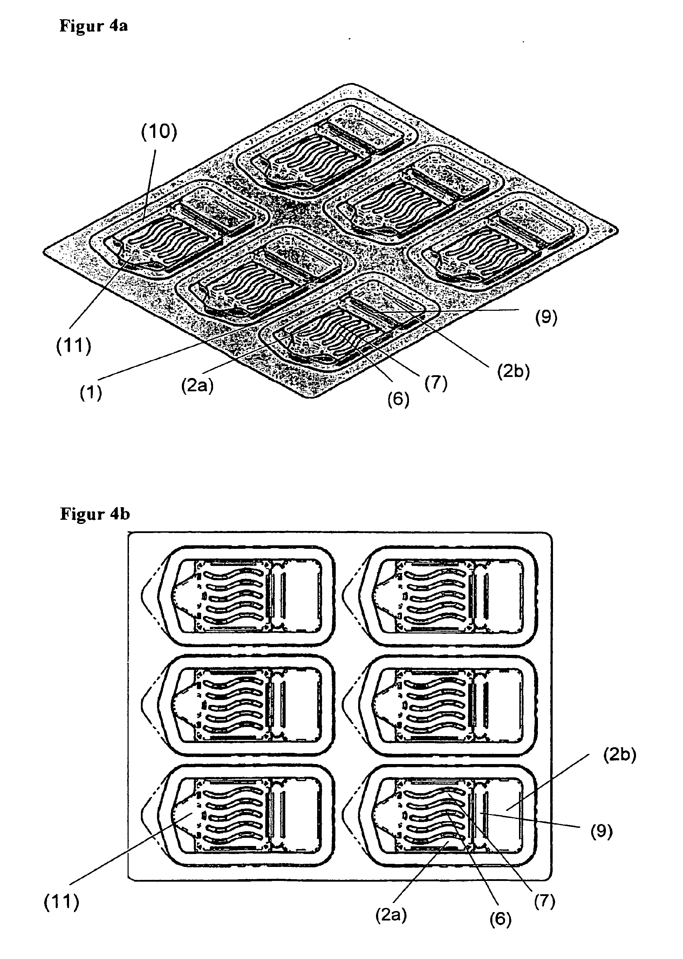

- Figur 4a:: zeigt ein Beispiel eines Basiselements mit mehreren Kavitäten (2a und 2b)
- Figur 4b:: zeigt ein Beispiel eines Basiselements mit mehreren Kavitäten (2a und 2b) in der Draufsicht dargestellt

### Beispiele

### Beispiel 1: Beschreibung der Herstellung der bioresorbierbaren Trägermaterialien (bioresorbierbare Wundauflagen)

Die Synthese der für die nachfolgend beschriebenen Beispiele verwendeten bioresorbierbaren Wundauflagen (Hydrolyse/Kondensation, Reaktiveindampfung und Reifung) wurde so wie in der WO2008086970A1 ausführlich beschrieben, durchgerührt. Die wesentlichen Prozessparameter sind nachfolgend zusammengefasst:

| | |
|---|---|
| Hydrolyse/Kondensation: | - in einem 2L-Rührkessel |
| | - bei molaren Verhältnissen |
| | - Reaktionsdauer ca. 19h |
| | - gerührt mit 150 U/min |
| | - 2h adiabate Fahrweise, anschließend isotherm bei 37°C |
| Reaktiveindampfung: | - in einem 2L-Rührkessel |
| | - Prozess: Eindampfen mittels Überschleierung von Steuerluft |
| | - isotherme Fahrweise bei 63°C |
| | - Reaktionsdauer ca. 5:20h |
| | - gerührt 60U/min |
| | - Viskosität am Ende (4°C; 10s-1): 0,95Pas |
| Reifung: | - in einem 500ml Reifebecher |
| | - Reifetemperatur 4°C |
| | - Dauer der Reifung (Polymerisation): 21d |
| Nach der Zwischenlagerung bei | -80°C erfolgte die Verspinnung: |
| | - während Verspinnung Temperierung des Sols auf ca. -12°C |
| | - Zuluft Spinnturm: Temperatur 25°C, Taupunkt 0°C |
| | - Verwendung einer 19-Düsenloch-Platte |
| | - Konditionierung aller Vliese in der Isolatorbox für ca. 40min |
| | - Zuschnitt des Vliese (5cm x 5cm) Vliese entspricht der in dieser Anmeldung besprochenen bioresorbierbaren Wundauflage |

### Beispiel 2: Verpackung der bioresorbierbaren Wundauflagen

Die Wundauflage wurde in einen erfindungsgemäßen Blister gelegt. Ein oder mehrere Adsorbentien wurden ebenfalls in eine dafür im Blister vorgesehene Vertiefung gelegt (bei Verwendung von 2 Adsorbens-Päckchen wurde das 2. Adsorbens-Päckchen lose dazugelegt). Der so gefüllte Blister wurde anschließend in einen vorgefertigten Aluminiumverbundfolienbeutel gepackt und unter leichtem Vakuum versiegelt. Aufgrund des Vakuums bildet der Beutel quasi einen Abschluss des Blisters, so dass die Wundauflage nicht herausfallen konnte. Bei den aufgeführten Verpackungen wurden außerdem folgende Variationen durchgeführt:
- Verpackung von der nach Beispiel 1 hergestellten bioresorbierbaren Wundauflage ohne ein Adsorbtionsmittel
- Verpackung von der nach Beispiel 1 hergestellten bioresorbierbaren Wundauflage mit 1 Päckchen 2,0g Silicagel
- Verpackung von der nach Beispiel 1 hergestellten bioresorbierbaren Wundauflage mit 1 Päckchen 2,0g Zeolith 4A
- Verpackung von der nach Beispiel 1 hergestellten bioresorbierbaren Wundauflage mit 1 Päckchen 2,0g Zeolith 13X
- Verpackung von der nach Beispiel 1 hergestellten bioresorbierbaren Wundauflage mit 1 Päckchen 2,0g Silikalit-1
- Verpackung von der nach Beispiel 1 hergestellten bioresorbierbaren Wundauflage mit 1 Päckchen 2,0g Silikalit-1 und 2,0g Zeoltih 4A

Adsorbensmaterialien können beispielsweise bei der Firma Multisorb bezogen werden.

### Beispiel 3: Degradationsverhalten der bioresorbierbaren Wundauflage

Für diesen Test wird ein Ausschnitt (ca. 20-30 mg) der bioresorbierbaren Wundauflage, welcher in einer Verpackung gemäß Beispiel 2 22 Tage gelagert wurde, eingewogen und in einen metallischen Siebeinsatz gegeben. Dieser wird dann in einen Behälter gegeben, in dem 500 ml 0,05 M Tris pH 7,4 Pufferlösung (Fluka 93371) bei 37°C thermostatisiert vorliegen. Zur Verbesserung des Stoffaustauschs rotiert das Siebkörbchen mit einer Drehzahl von 50 rpm. Während der gesamten Messzeit wird die Pufferlösung bei 37°C temperiert. Dabei findet eine gewisse jedoch geringfügige Durchmischung des Behälterinhalts statt. Der Degradationsverlauf wird mittels Si-Analyse der flüssigen Phase verfolgt. Dabei werden in Zeitabständen Proben (ca. 1 mL Lösung) innerhalb einer Zeitperiode von 24 Stunden genommen und per ICP-OES auf den Si-Gehalt analysiert. Die Ergebniswerte in µg/mL werden mit dem Faktor 1,070 in mg absolut umgerechnet (60,09/28,09 x 500/1000). Die Messwerte in mg absolut werden grafisch gegen die Messzeitpunkte aufgetragen. Nur über den linearen Bereich des Anstiegs wird eine lineare Regression durchgeführt. Die Steigung entspricht dem Abbau in mg/h. Unter Berücksichtigung der Einwaage wird der relative Abbau in Prozent/h berechnet ([mg/h] / [mg] * 100 = [%/h]). Für die Wiederfindung [%]) wird der mg Absolutwert mit dem theoretischen Si-Gehalt (0,38) multipliziert. Tabelle 1 zeigt die Ergebnisse des Degradationstests für verschiedene Verpackungen aus Beispiel 2.

**Tabelle 1: Resultat des Degradationstests**

| **Verpackung** | **Lagerungszeit bis zum Test** | **Rate [%/h])** | **Wiederfindung [%])** | **Beobachtung** |
|---|---|---|---|---|
| ohne Adsorbens | 22 Tage | 2,8 | 67,2 | leichter Rückstand |
| Silicagel | 28 Tage | 10,2 | 91,7 | vollständig gelöst, keine Rückstände |
| Zeolith 4A | 22 Tage | 3,3 | 76,8 | vollständig gelöst, keine Rückstände |
| Zeolith 13X | 22 Tage | 2 | 47,3 | nur teilweise gelöst |
| Silikalit-1 | 28 Tage | 7,4 | 90,4 | vollständig gelöst, keine Rückstände |
| Silikalit-1 + Zeolith 4A | 28 Tage | 9,1 | 91,8 | vollständig gelöst, keine Rückstände |

Bioresorbierbare Wundauflagen, die in einer Verpackung ohne Adsorbens gelagert wurden, degradieren deutlich langsamer als diejenigen, die in einer Verpackung mit Silicagel, Zeolith 4A oder Silikalit-1 + Zeolith 4A aufbewahrt wurden. Verzögerte Resorbierbarkeit bzw. eine unvollständige Resorbierbarkeit kann *in vivo* dazu führen, dass Rückstände im Gewebe zurückbleiben, die über längere Zeitraum ungewünschte Entzündungsreaktionen hervorrufen bzw. zu einer nicht physiologischen Wundheilung führen können.

Es wurden die Gasphasenkonzentrationen von Wasser und Ethanol in den Verpackungen aus Beispiel 2 nach 3, 11 und 25 Tagen bestimmt. Im Fall der Verpackung ohne Adsorbens lag die Konzentration von Wasser um einen Faktor zwischen 1,8 (3 Tage, Silikalit-1) und 6 (11 Tage, Silicagel) höher als in den Verpackungen mit einem oder mehreren Adsorbentien.

Im Fall der Verpackung ohne Adsorbens lag die Konzentration von Ethanol sogar um einen Faktor über 1000 höher als im Fall der Verpackung mit Silikalit-1 + Zeolith 4A nach 25 Tagen.

Die Adsorbentien führen damit zu einer Verringerung der Konzentration von Wasser und/oder Ethanol in der Gasphase in der Verpackung.

Es wurden mittels NIR-Spektroskopie (Auswertung der Absorptionsbande bei 5250 cm⁻¹) die Wassergehalte der Trägermaterialien aus den Verpackungen des Beispiels 2 nach 25 Tagen Lagerung in der Verpackung gemessen.

Der Wassergehalt eines Trägermaterials, gelagert in einer Verpackung mit Silkalit-1 als Adsorbens betrug im Mittel 4,5 Gew.-%, der Wassergehalt eines Trägermaterials, gelagert in einer Verpackung mit Silicagel als Adsorbens betrug im Mittel 3,2 Gew.-%, der Wassergehalt eines Trägermaterials, gelagert in einer Verpackung mit Silkalit-1 + Zeolith 4A als Adsorbentien betrug im Mittel 1,1 Gew.-%.

Es wurden die Ethoxygehalte der Trägermaterialien der Beispiele 2 nach einer Lagerung in der Verpackung von 1 Tag, von 8 Tagen und von 22 Tagen gemessen.

Bei allen Trägermaterialien zeigte sich eine Abnahme des Ethoxygehalts im Verlauf der Zeit - mit Ausnahme der Trägermaterialien, die zusammen mit Silkalit-1 + Zeolith 4A als Adsorbentien gelagert wurden. Bei den letztgenannten Trägermaterialien war auch nach 25 Tagen innerhalb der Messgenauigkeit keine zeitliche Veränderung zu beobachten. Die Trägermaterialien, die in einer Verpackung mit einem Wasser-Adsorbens (Zeolith 4A) und einem Ethanol-Adsorbens (Silikalit-1) gelagert wurden, zeigen eine Biodegradierbarkeit auch noch nach vielen Monaten.

## Patentansprüche

1. Ein Produkt umfassend
a) eine Verpackung aus:
1) einem mechanisch stabilen Basiselement (1) mit wenigstens einer nach wenigstens einer Seite hin offenen Kavität (2a), zur Entnahme wenigstens eines in der Kavität (2a) bioresorbierbaren Trägermaterials,
2) zumindest einem Adsorbens,
3) zumindest einer Siegelfolie (3), die zumindest die Öffung der Kavität, welche ein darin eingebertetes bioresorbierbares Trägermaterial enthält, verschließt,
4) und optional einem Beutel (4) aus Aluminium- oder Aluminiumverbundfolie, welcher das Basisclement (1) mit Siegeltolie (3) komplett umhüllt und verschließt, und
b) ein bioresorbierbares Trägermaterial, welches sich zumindest in einer Kavität (2a) des mechanisch stabilen Basiselements (1) der Verpackung befindet,
dadurch gekenngeichnet, dass
sich das bioresorbierbare Trägermaterial für die Geweberegeneration eignet und ausgewählt ist aus der Gruppe bestehend aus Kieselgelfasern und Kiselgelvliesen, und dass
sich in der Verpackung mindestens ein Adsorbens für Wasser und mindestens ein Adsorbens für Ethanol befindet.

2. Produkt nach Anspruch 1, **dadurch gekennzeichnet, dass** das mechanisch stabile Basiselement (1) zusätzlich eine Metallfolie aus Aluminium- oder Aluminiumverbundfolie umfasst.

3. Produkt nach Anspruch 1 oder 2, dadurch gekenntzeichnet, dass das mechanisch stabile Basiselement (1) der Verpackung eine zweite Kavität (2b) zur Aufnahme mindestens eines Adsorbens umfasst.

4. Produkt nach Anspruch 3, **dadurch gekennzeichnet, dass** zumindest eine Siegelfolie (3) auch die Öffnung der Kavität (2b), welche mindestens ein darin eingebettetes Adsorbens enthält, verschließt.

5. Produkt nach einem der Ansprüche 1 bis 4, **dadurch gekennzeichnet, dass** das bioresorbierbare Trägermaterial zur Geweberegeneration eine Wundauflage ist.

6. Produkt nach Anspruch 5, **dadurch gekennzeichnet, dass** die Kavität (2a) zur Entnahme wenigstens einer in der Kavität (2a) angeordneten Wundauflage, in der Höhe, Breite und Länge so ausgestaltet ist, dass ein Verrutschen der Wundauflage in der Kavität in eine der drei Dimensionen im Wesentlichen verunmöglicht wird.

7. Produkt nach einem der Ansprüche Soder 6 umfassend eine Verpackung und einer sich in der Verpackung befindlichen Wundauflage, **dadurch gekennzeichnet, dass** die Kavität (2a) des mechanisch stabilen Basiselements der Verpackung zur Entnahme wenigstens einer in der Kavität (2a) angeordneten Wundauflage eine Bodenplatte (5) mindestens einen Kanal (6) zur Aufnahme von Benetzungsnussigkeit und mindestens einem Steg (7) zur Drainage der Benetzungsflüssigkeit aufweist.

8. Produkt nach einem der vorhergehenden Ansprüche, **dadurch gekennzeichnet, dass** die Schichtdicke des Beutels (4) aus Aluminium- oder Aluminiumverbundfolie der Verpackung mindestens 16 µm beträgt.

9. Produkt nach einem der Ansprüche 3 bis 8, **dadurch gekennzeichnet, dass** die Kavität (2b) zur Aufnahme eines Adsorbens auf mindestens einer Seite des Basiselement offen ist und die Siegelfolie die Öffnungen der wenigstens zwei nach wenigstens einer Seite hin offenen Kavitäten (2a und 2b) des Basiselements unter Ausbildung von Holräumen verschließt und sich die Siegelfolie von einem Anwender nur zur Entnahme wenigstens eines in der Kavität (2a) angeordneten bioresorbierbaren Trägermaterials abziehen lässt.

10. Produkt nach einem der Ansprüche 3 bis 9, dadurch gekennzeichet, dass die Kavität (2b) zur Aufnahme des Adsorbens einen durch das Basiselement (1) selber geformten abgeschlossenen Hohlraum bildet.

11. Verfahren zur Herstellung eines Produkts nach einem der Ansprüche 1 bis 10 bei der
a) in eine Verpackung mit einem mechanisch stabilen Basiselement (1) mit wenigstens einer nach wenigstens einer Seite hin offenen Kavitäten (2a), zur Entnahme wenigstens eines in der Kavität (2a) bioresorbierbaren Trägermaterials wenigstens ein bioresorbierbares Tragermaterial in die Kavität (2a) eingelegt wird,
b) eine Siegelfolie (3) zumindest diejenige Kavität, welche ein darin eingebettetes bioresorbierbares Trägermaterial enthält, derart verschlossen wird, dass entweder zwischen der Siegelfolie (3) und einem Teil des Basiselements (1) eine die Kavität vollständig umschließende heißversiegelte Verbindung entsteht oder zwischen Teilen der Siegelfolie (3) selber eine heißversiegelte Verbindung entsteht, so dass das Basiselement und das bioresorbierbare Trägermaterial sich nach dem Verschließen in der Siegelfolie (3) befinden,
c) optional das mechanisch stabile Basiselement mit der durch die Siegelfolie (3) verschlossenen Kavität mit einem Beutel (4) aus Aluminium- oder Aluminiumverbundfolie, weicher das Basiselement (1) mit Siegelfolie (3) komplett umhüllt, versiegelt wird, und
d) mindestens ein Adsorbens für Wasser und mindestens ein Adsorbens für Ethanol vor dem Versiegeln in Schritt b) oder c) zur Verpackung dazugegeben wird.

12. Verfahren nach Anspruch 11, **dadurch gekennzeichnet, dass** das mechanisch stabile Basiselement (1) aus Schritt a) zusätzlich mit einer Metallfolie aus Aluminium- oder Aluminiumverbundfolie ausgestattet wird.

13. Verwendung eines Produktes der Ansprüche 1 bis 10 zum Verpacken und Lagern von bioresorbierbaren Trägermaterialien.

## Claims

1. A product comprising
a) a packaging of:
1) a mechanically stable base element (1) having at least one cavity (2a) open towards at least one side, for the removal at least of one bioabsorbable carrier material arranged in the cavity (2a),
2) at least one adsorbent,
3) at least one sealing foil (3) that closes at least the opening of the cavity, which contains a bioabsorbable carrier material embedded therein,
4) and optionally a pouch (4) made of aluminium foil or laminated aluminium foil, which completely surrounds and closes the base element (1) with sealing foil (3), and
b) a bioabsorbable carrier material, which is situated at least in one cavity (2a) of the mechanically stable base element (1) of the packaging, **characterized in that** the bioabsorbable carrier material is suitable for tissue regeneration and is selected from the group consisting of silica gel fibres and silica gel webs, and **in that** at least one adsorbent for water and at least one adsorbent for ethanol is situated in the packaging.

2. Product according to Claim 1, **characterized in that** the mechanically stable base element (1) additionally comprises a metal foil of aluminium foil or laminated aluminium foil.

3. Product according to Claim 1 or 2 **characterized in that** the mechanically stable base element (1) of the packaging comprises a second cavity (2b) for receiving at least one adsorbent.

4. Product according to Claim 3, **characterized in that** at least one sealing foil (3) also closes the opening of the cavity (2b), which contains at least one adsorbent embedded therein.

5. Product according to one of Claims 1 to 4, **characterized in that** the bioabsonbable carrier material for tissue regeneration is a wound dressing.

6. Product according to Claim 5, **characterized in that** the cavity (2a) for the removal at least of one wound dressing arranged in the cavity (2a) is designed in height, breadth and length so that slipping of the wound dressing in the cavity in one of the three dimensions is essentially made impossible.

7. Product according to one of Claims 5 and 6 comprising a packaging and a wound dressing situated in the packaging, **characterized in that** the cavity (2a) of the mechanically stable base element of the packaging has, for the removal at least of one wound dressing arranged in the cavity (2a), a floor plate (5), at least one channel (6) for receiving wetting fluid and at least one bridge (7) for the drainage of the wetting fluid.

8. Product according to one of the preceding claims, **characterized in that** the layer thickness of the pouch (4) of aluminium foil or laminated aluminium foil of the packaging is at least 16 µm.

9. Product according to one of Claims 3 to 8, **characterized in that** the cavity (2b) for receiving an adsorbent is open on at least one side of the base element and the sealing foil closes the openings of the at least two cavities (2a and 2b) of the base element which are open towards at least one side with formation of cavities and the sealing foil can be peeled off by a user only for the removal at least of one bioabsorbable carrier material arranged in the cavity (2a).

10. Product according to one of Claims 3 to 9, **characterized in that** the cavity (2b) for receiving the adsorbent forms a closed-off void formed by the base element (1) itself.

11. Process for the production of a product according to one of Claims 1 to 10 in which
a) in a packaging containing a mechanically stable base element (1) with at least one cavity (2a) open towards at least one side, at least one bioabsorbable carrier material is inserted into the cavity (2a) for the removal at least of one bioabsorbable carrier material in the cavity (2a),
b) a sealing foil (3) at least the cavity, which contains a bioabsorbable carrier material embedded therein, is closed such that either a heat-sealed bond completely surrounding the cavity results between the sealing foil (3) and a part of the base element (1) or a heat-sealed bond results between parts of the sealing foil (3) itself, so that the base element and the bioabsorbable carrier material are situated in the sealing foil (3) after closure,
c) optionally the mechanically stable base element is sealed with the cavity closed by the sealing foil (3) with a pouch (4) of aluminium foil or laminated aluminium foil, which completely surrounds the base element (1) with sealing foil (3), and
d) at least one adsorbent for water and at least one adsorbent for ethanol is added to the packaging before the sealing in step b) or c).

12. Process according to Claim 11, **characterized in that** the mechanically stable base element (1) from step a) is additionally equipped with a metal foil of aluminium foil or laminated aluminium foil.

13. Use of a product of Claims 1 to 10 for the packaging and storing of bioabsorbable carrier materials.

## Revendications

1. Produit comprenant
a) un emballage, composé de
1) un élément de base mécaniquement stable (1) avec au moins une cavité (2a) ouverte vers au moins un côté, pour prélever au moins un matériau de support biorésorbant dans la cavité (2a),
2) au moins un adsorbant,
3) au moins un film de scellement (3), qui ferme au moins l'ouverture de la cavité, qui continent un matériau de support biorésorbant logé dans celle-ci,
4) et éventuellement un sachet (4) en film d'aluminium ou de composite d'aluminium, qui entoure complètement et enferme l'élément de base (1) avec le film de scellement (3), et
b) un matériau de support biorésorbant, qui se trouve au moins dans une cavité (2a) de l'élément de base mécaniquement stable (1) de l'emballage,
**caractérisé en ce que** le matériau de support biorésorbant convient pour la régénération de tissus et est sélectionné dans le groupe constitué des fibres de gel de silice et des toiles de gel de silice, et **en ce qu'**il se trouve dans l'emballage au moins un adsorbant pour l'eau et au moins un adsorbant pour l'ethanol.

2. Produit selon la revendication 1, **caractérisé en ce que** l'élément de base mécaniquement stable (1) comprend en outre un film métallique en film d'aluminium ou de composite d'aluminium.

3. Produit selon la revendication 1 ou 2, **caractérisé en ce que** l'élément de base mécaniquement stable (1) de l'emballage comprend une deuxième cavité (2b) destinée à contenir au moins un adsorbant.

4. Produit selon la revendication 3, **caractérisé en ce qu'**au moins un film de scellement (3) ferme en outre l'ouverture de la cavité (2b), qui contient au moins un adsorbant logé dans celle-ci.

5. Produit selon l'une quelconque des revendications 1 à 4, **caractérisé en ce que** le matériau de support biorésorbant est un pansement pour la régénération de tissus.

6. Produit selon la revendication 5, **caractérisé en ce que** la cavité (2a) pour prélever au moins un pansement disposé dans la cavité (2a) est configurée, en hauteur, largeur et longueur, de telle manière qu'un glissement du pansement dans la cavité dans l'une des trois dimensions soit rendu essentiellement impossible.

7. Produit selon l'une des revendications 5 ou 6 comprenant un emballage et un pansement se trouvant dans l'emballage, **caractérisé en ce que** la cavité (2a) de l'élément de base mécaniquement stable de l'emballage pour prélever au moins un pansement (5) disposé dans la cavité (2a) présente au moins un canal (6) destiné à contenir un liquide de mouillage et au moins une nervure (7) pour le drainage du liquide de mouillage.

8. Produit selon l'une quelconque des revendications précédentes, **caractérisé en ce que** l'épaisseur de couche du sachet (4) en film d'aluminium ou de composite d'aluminium de l'emballage vaut au moins 16 µm.

9. Produit selon l'une quelconque des revendications 3 à 8, **caractérisé en ce que** la cavité (2b) destinée à contenir un adsorbant est ouverte sur au moins un côté de l'élément de base, et le film de scellement ferme les ouvertures des au moins deux cavités (2a et 2b) de l'élément de base ouvertes vers au moins un côté en formant des espaces creux et le film de scellement ne peut être enlevé par un utilisateur que pour prélever au moins un matériau de support biorésorbant disposé dans la cavité (2a).

10. Produit selon l'une quelconque des revendications 3 à 9, **caractérisé en ce que** la cavité (2b) destinée à contenir l'adsorbant forme un espace creux fermé formé par l'élément de base lui-même (1).

11. Procédé de fabrication d' un produit selon l' une quelconque des revendications 1 à 10, dans lequel
a) dans un emballage comportant un élément de base mécaniquement stable (1) avec au moins une cavité (2a) ouverte vers au moins un côté, pour prélever au moins un matériau de support biorésorbant dans la cavité (2a), on dépose au moins un matériau de support biorésorbant dans la cavité (2a),
b) on ferme au moyen d'un film de scellement (3) au moins la cavité, qui contient un matériau de support biorésorbant logé dans celle-ci, de telle manière qu'il se forme entre le film de scellement (3) et une partie de l'élément de base (1) une liaison soudée à chaud entourant entièrement la cavité ou qu'il se forme une liaison soudée à chaud entre des parties du film de scellement (3) lui-même, de telle manière que l'élément de base et le matériau de support biorésorbant se trouvent dans le film de scellement (3) après la fermeture,
c) éventuellement l'élément de base mécaniquement stable est scellé à la cavité fermée par le film de scellement (3) avec un sachet (4) en film d'aluminium ou en film composite d'aluminium, qui enveloppe complètement l'élément de base (1) avec le film de scellement (3), et
d) on ajoute au moins un adsorbant pour l'eau et au moins un adsorbant pour l'ethanol à l'emballage avant le scellement à l'étape b) ou c).

12. Procédé selon la revendication 11, **caractérisée en ce que** l'élément de base mécaniquement stable (1) de l'étape a) est en outre muni d'un film métallique constitué d'un film d'aluminium ou d'un film composite d'aluminium.

13. Utilisation d'un produit selon les revendications 1 à 10 pour l'emballage et le stockage de matériaux de support biorésorbants.
